# EUROPEAN PATENT APPLICATION

(11) **EP 4 765 148 A1**
(43) Date of publication of application: **24.06.2026**
(21) Application number: 24221151.4
(22) Date of filing: 18.12.2024
(51) Int. Cl.: G16H 30/40, G16H 70/20

(54) **METHOD AND SYSTEM FOR OPTIMIZING AN EXAMINATION**

(71) Applicant: Koninklijke Philips N.V., 5656 AE Eindhoven (NL)
(72) Inventor: VON BERG, Jens, 5656 AG Eindhoven (NL); GRASS, Michael, 5656 AG Eindhoven (NL); VOGTMEIER, Gereon, 5656 AG Eindhoven (NL); WEIß, Steffen, 5656 AG Eindhoven (NL); HELLE, Michael Günter, 5656 AG Eindhoven (NL); FORTHMANN, Peter, 5656 AG Eindhoven (NL)
(74) Representative: Philips Intellectual Property & Standards

(57) **Abstract**

The invention concerns a computer-implemented method for optimizing an examination, wherein the method comprises the steps of instructing the patient by displaying instructions via a user interface for simulating the examination; receiving image data comprising information about at least one portion of the patient via an optical sensor; analyzing, by a processor, the image data from the patient when the patient has been instructed; determining visible patient properties based on the analyzed image data; providing the visible patient properties to an external examination unit; and adapting a workflow of the examination based on the determined visible patient properties. The invention further concerns a system for optimizing an examination.

## Description

### FIELD OF THE INVENTION

The invention relates to the field of optimizing an examination of a patient.

More specifically, the invention relates to the field of patient preparation before an examination for optimizing the examination.

### BACKGROUND OF THE INVENTION

For an upcoming examination, for example diagnostic examination or treatment procedures, patients often get information in written form for them to prepare at home. This should help the patient to adjust to the examination and to run the examination smoothly. When a patient should participate in an examination or diagnostic procedure, there are often delays in examination procedure because the procedure for arranging the patient or technical parameters have to be adjusted to the individual patient. Further, there may arise extraordinary conditions that need specific consideration, for example when a patient may not be able to communicate or the like.

### SUMMARY OF THE INVENTION

Therefore, there exists a need for optimizing an examination of a patient and for gaining any information available about the patient ahead of the examination, i.e. the acquisition, which could help in adapting the procedure for the examination and/or presetting conditions.

An object of the invention is to address at least some of the above challenges, in particular to provide an effective and improved workflow for an examination of a patient. In particular, it is the objection of the invention to prepare a patient and/or obtaining data about the patient for optimizing the examination.

The object of the present invention is solved with the subject matter of the independent claims, wherein further embodiments are incorporated in the dependent claims.

According to a first aspect of the invention, a computer-implemented method for optimizing an examination is provided. The method comprises the steps of instructing the patient by displaying instructions via a user interface for simulating the examination; receiving image data comprising information about at least one portion of the patient via an optical sensor; analyzing, by a processor, the image data from the patient when the patient has been instructed; determining visible patient properties based on the analyzed image data; providing the visible patient properties to an external examination unit; adapting a workflow of the examination based on the determined visible patient properties.

The order of the steps may not be limited to the order of listing. Instead, the steps may be performed in a different order as listed and/or some or all of the steps of the method may be performed simultaneously or at least in an overlapping manner.

In the context of the present invention, the term "examination" may be understood to describe a procedure the patient has to undergo, such as an X-ray examination. The term "examination" may not be limited to X-ray examination, as other examination for example magnetic resonance tomography (MRT) examinations, ultrasound examination, sonography examinations, computed tomography (CT) examinations may also be applicable.

In the context of the present invention, the method is for example a computer implemented method. This means that for example all steps or merely some steps of the method as described herein may be performed by a computer. For example, at least one computer in a handheld device such as a mobile phone. The computer may be used for data processing, and/or the computer may be operated in such a way that it performs the method of the present invention, or at least one or some steps of the method. The computer may comprise at least one processor which is configured to perform one, some or all of the method steps of the present invention. The processor may be configured to process the data electronically.

In the context of the present invention, the term "workflow" may be understood to describe a medical workflow which may be performed during an examination. On the other hand, the workflow may also comprise steps which should be performed before the examination. The workflow may comprise different steps which may be performed in a typical order depending on the respective examination. For example, a step of the workflow may be at least one of the following steps capturing a medical image of the patient during the examination, positioning a patient, determining imaging parameters such as parameter settings for an imaging device, determining and choosing specific radiographic views, language requirements for guiding the patient and the like.

The present invention may provide instructions, which may be visible instructions displayed using the user interface, acoustical instructions may also be used or any combination thereof. For instance, the instructions may show an artificial avatar of a human body or of parts of the human body which should guide the patient to a specific examination position, posture or the like. Further, it may be possible that arrows are displayed for indicating movement directions or a position for the body or at least for parts of the body of the patient. The instructions may be displayed to a user, wherein the user may be the patient itself, or the user may be a care person, for instance a care worker or a nurse in a nursing home, retirement home. If the user is not the patient, the patient may be instructed indirectly via the user.

The present invention may provide a user interface for interacting with the patient, such as instructing the patient and/or receiving input data from the patient via the user interface. On the other hand, the user interface may be used for displaying the instructions to another person, such as a care worker or a nurse which in turn provides the instructions to the patients. The user interface may be configured to support the user, i.e. the patient or any other patient support person, in adjusting a patient position, or at least a position of a part of the patient (body part) to a target position for the planned examination. According to an exemplary embodiment of the invention, the user interface may be formed from a display, for example a touch sensitive display. Further, the user interface may be part of a mobile communication unit. Furthermore, the user interface may comprise an input unit, such as keypad, and/or a voice interface of the mobile communication unit. For example, the mobile communication unit may be a handheld device such as a mobile phone, a smartphone, or a tablet. Moreover, the optical sensor may be part of the user interface. In particular the optical sensor may be part of the mobile communication unit for example as a camera of the mobile phone. The optical sensor may be a 2D or 3D camera configured to receive user inputs wire the camera and/or configured to capture image data of at least parts of the patient or of the overall patient.

In the context of the present invention, the term "visible patient properties" may be understood to describe features of the patient which can be derived from the received image data. This means every data obtained by the image sensor could be described as visible patient properties. The visible patient properties may be properties relevant for the planned examination and which may differ from patient to patient, such that an individual adaptation may be necessary in the workflow of the examination. For example, if the patient is recognized via the optical sensor, the body or at least one or all body parts of the patient may be recognized. Additionally, any other element may be captured via the sensor and may be received as image data, for instance the position of the patient, any support devices used by the patient. A non-limiting list of visible patient properties may be a position of body parts of the patient, a posture of at least one body part of the patient, a posture of the whole patient (such as sitting, standing, standing inclined or the like), flexing or rotating joints or bending muscles, different postures of different body parts in comparison, support devices (a wheel chair, a chair/seat, a rollator, a crutch).

The received information about the patient may be at least information about a portion of the patient. A portion may be at least one body part of the patient, such as a joint, a hand, a leg, an arm, a head, or it may be a plurality of body parts, or it may be the whole body of the patient.

The processor may be configured to process and/or analyze the image data captured by the optical sensor in a variety of ways in order to understand, for example their content or extract geometric information about the patient. For example, the processor may be configured to perform computer vision by applying object recognition, measurement of geometric structures of objects and movements or similar processes. The processor may be in communication with the external examination unit for instance via wireless communication, such that the processor is able to transfer the received image data and also the analyzed/determined patient properties (visible or non-visible per user input).

As an additional embodiment, an acoustic sensor may be used for determining non-visible patient properties, such as spoken words which may indicate any hindrance of the patient when performing according to the instructions.

In other words, the method according to the first aspect provides a computer implemented method, which for instance may be implemented as a mobile phone application, which is configured to provide instructions to a patient on how to practice a procedure, e.g. a positioning for a medical image, for example an X-ray image, acquisition. The method performs steps and may be configured to visually observe the patient by using the optical sensor, while practicing the procedure, i.e. practicing according to the instructions. Further the method comprises steps and may be configured to collect data about the patient by, for example, determining individual patient properties. In particular, the method may be provided to elderly patients at home or elderly care to prepare for a scheduled X-ray examination by practicing the pose. While practicing the instructions, and/or while instructing the patient the method may collect data, the patient properties, such as size, weight, mobility, cognitive skills, spoken language, general health status. This data may be used to preset, automate, and/or individualize the actual examination process. The examination process may be preset and/or individualized in the examination unit, which may be for example a department for scheduling staff and material. The patient properties can be received before the examination has to be performed, such that the workflow can be adapted prior to the examination, what in turn reduces delays before the examination of the patient. All information can be obtained by one single method and can be transferred all together without the need to receive it separately. Accordingly, the examination procedure can be simplified.

It should be noted that any feature, function and/or element described in the following with reference to the system for optimizing an examination equally applies to the method, and vice versa. Accordingly, any feature, function, step and/or element described in the following with reference to one aspect of the present disclosure equally applies to any other aspect of the present disclosure.

According to an exemplary embodiment of the invention, the method may further comprise the steps of applying a 3D anatomical model to the image data; wherein the step of determining patient properties may further comprise adapting the 3D anatomical model to the patient using the determined visible patient properties for generating a patient specific 3D anatomical model wherein the patient specific 3D anatomical model may be provided to the external examination unit. This model may be an explicit representation of the body and its pose with edges representing limbs and nodes representing joints parameterized by lengths and angles. It also may be a rather implicit model where these pose and shape parameters are represented as weighted sums distributed over a number of model parameters.

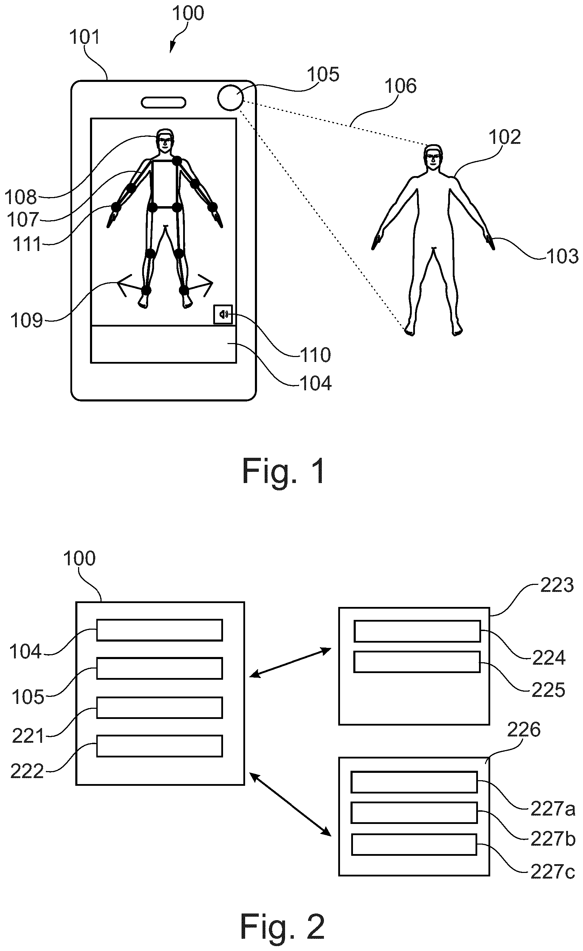

The steps performed by the method may be computer vision steps. The processor may be configured to analyze the image data for determining specific visual patient properties, such as patient size and weight parameters. For example, the size of each body part may be determined by deriving geometrical parameters from the image data. After this determination variable size parameters for each body part may be available as visible patient properties for further processing. The size and weight parameters may be used for applying an individualized 3D body model, which is also described in Fig. 1. The individualized model may be used for adapting, for example the examination parameters.

According to an exemplary embodiment of the invention, the step of determining patient properties may further comprise generating a mobility protocol of the patient based on the analyzed image data and/or the visible patient properties, wherein the mobility protocol may comprise information about an overall mobility of the patient derived from the image data and/or the visible patient properties received during performing the instructions by the patient. This protocol may contain a flexion angle that the patient is able to execute in the training or whether the patient was able to take a required pose or not. The degree of the patient meeting pose requirements by its actual pose can be calculated by the parameters of the model reflecting the actual pose of the patient compared to the given parameters of an ideal pose. The ideal pose may be predetermined by a medical professional, wherein it may depend on the examination to be performed.

In other words, the image data may be analyzed, for example whether support devices are used. This means the processor may be configured to perform an image analysis and thereby analyze different image features than the patient. Accordingly, the processor may not necessarily determine the visible patient properties, instead it is analyzed whether further elements can be derived from the image data such as any kind of support devices (wheelchair, rollator, or crutch). Differently speaking, for determining the mobility protocol the image data are analyzed with respect to different data/features other than the patient. Further, the image data are analyzed with respect to how the patient is able to perform the instructions and how the patient is able to adjust to a specific examination position/posture/movement. Therefore, for determining a mobility protocol a patient itself must not be analyzed but for immobility caused by injuries or pain the visible patient properties are relevant. The overall mobility of the patient may be understood to describe a level of the mobility of the patient, and how the patient is able to participate in the examination. For example, 100% overall mobility may indicate that the patient is able to follow the instructions and perform the instructions correctly such that there exists no hindrance for performing planned examination. The other levels of overall mobility may be determined based on the analyzed image data and or the visible patient properties. For example, when the patient uses a wheelchair which is determined with the patient properties from the image data, the overall mobility may be at a level of 50%. On the other hand, when an immobility is caused due to a disease or due to pain, the level of the overall mobility may be determined based thereon how the patient is able to perform the instructions. The overall mobility of the patient may be obtained by comparing the image data of the performing patient with and correct performed instruction. The correct performed instruction may be a predetermined input. A perfect performed instruction may be understood to describe instruction of taking a position or performing a movement by patient which is not disabled or in pain and where the patient is able to stand in the position or perform the movement correctly.

According to an exemplary embodiment of the invention, the step of determining patient properties may further comprise determining at least one of a size of the patient, a weight of the patient, a disease of the patient, or a physical malfunction of the patient from the image data, or from the presence of immobility devices like a cast, some prothesis, wheelchair etc.

For instance, from the image data a disease such as scoliosis, a stroke, and/or a physical disability may be derived. If the instructions instruct the patient to perform specific joint posture of the respective body part, it may be derived from the image data, whether the joint posture can be reached by the patient or not. Depending on the outcome, for example if the joint posture may not be reached, the examination workflow may be adapted by using different postures for the patient. Furthermore, the size and/or the weight of the patient may be used for adapting a dose setting of the radiation emitting device for the respective imaging procedure. Moreover, the determined size and/or weight of the patient may be used for adapting the 3D patient model described with the exemplary embodiment of the invention.

According to an exemplary embodiment of the invention, the method may further comprise the steps of providing feedback to the patient via the user interface, whether the instructions have been performed correctly, and may comprise additionally displaying the feedback via the user interface.

The feedback to the patient may be provided by the user interface directly during performing the instructions such that the patient is able to realize whether he has to readjust or perform further movements for achieving the correct position, the posture and/or movements which is instructed. Furthermore, the feedback may comprise a step of comparing the performed instruction with expected correct instruction and determining the deviation of the performed instruction from the correct instruction. For example, whether the position is correct, or how the 3D model differs from the ideal 3D model for respective examination. This helps to adapt the correct patient position for the examination.

According to an exemplary embodiment of the invention, the method may further comprise the step of receiving an input from the patient about non-visible patient properties, wherein the non-visible patient properties are at least one of an age of the patient, a patient language, date of the examination, time of the examination, or a disease of the patient.

Further examples of non-visible patient properties may be acute pain regions, and/or pre-existing illnesses. On the other hand, the non-visible patient properties may be preferences input from the patient. For example, which language the patient speaks or prefers, if multilingual. The non-visible patient properties may be obtained by requesting input from the patient, for example before the instruction will be displayed via the user interface. Hence, the patient may complete different questions related to the non-visible patient properties, wherein the questions may be displayed via the user interface to the user. It may also be described as that the method further comprises steps of interviewing the patient. Differently speaking, the interface may be configured to provide questions to the patient, which have to be answered by the patient such that further information about the patient may be gathered. For example, whether the patient has allergies, which is important when planning an examination comprising contrast mediums. This in turn may be considered for adapting the workflow by using different contrast mediums.

According to an exemplary embodiment of the invention, the processor may be in communication with at least one software app, wherein the method may comprise the further step of receiving visible and/or non-visible patient properties from the software app.

In other words, the processor may be in communication with an interface to other non-medical data like fitness apps to import data. The at least one software app may be any further software app different to the app using the computer-implemented method according to the invention. The further software app may be implemented in the same computer, in particular in the same mobile communication device as the computer implemented method according to the invention. Therefore, the processor may be configured to control at least the data received from the further software app and may be configured to process the received data from the further software app. Examples for other non-medical data may comprise avatars of social media, garment avatar providing individual patient shape used in online shopping apps. This data may be used to optimize the diagnostic procedure.

According to an exemplary embodiment of the invention, the method may further comprise the step of receiving information from the external examination unit for further instructions to be displayed to the patient via the user interface, after the workflow of the examination has been adapted based on the previously determined visible patient properties. In other words, when the examination unit has adapted the workflow based on the visible patient properties, there may be the case that further amendments of the workflow may be necessary. For example, when realizing that with the present patient properties, the workflow may not be sufficient. The examination unit may then be able to provide further instructions, which may be different instruction than before. These further instructions may be provided and displayed to the patient. Hence, as an additionally step the method may comprise the steps of displaying the further instructions to the patient, receiving further image data comprising information about the at least one portion of the patient via the optical sensor (based on the further (new) instructions); repeat analyzing the image data from the patient when displaying the further instructions; determining further visible patient properties based on the analyzed image data; providing the further visible patient properties to the external examination unit; and repeating adapting of the workflow of the examination based on the determined further visible patient properties. This may serve to adapt the workflow of the examination until it may be determined that a sufficient and feasible workflow can be carried out with the individual patient.

According to an exemplary embodiment of the invention, the instruction may comprise at least one of the following steps of displaying single images for positioning the at least one portion of the patient in a position for the examination, and/or displaying image sequences for performing a movement of the at least one portion of the patient for the examination. It may also be possible that a combination of displaying at least one image and displaying image sequences are used. The respective image or image sequence may depend on the examination to be performed. For example, the examination may be a hand X-ray examination. The patient may be instructed via an image showing a respective hand in a specific position. Further, a model of the hand of the patient may be derived from the image data received from the patient and may be adapted to the image of the hand. The patient may be guided via instructions, for example arrows indicating how to position fingers, joints of the hand. A correct position may be achieved when the image of the hand overlaps in the correct position with the model hand of the patient. The same procedure may be applied using image sequences, which can be used for walking analysis of the patient.

According to an exemplary embodiment of the invention, the step of adapting a workflow of the examination may further comprise adapting examination parameters, wherein the examination parameters may be at least one of an imaging parameter, an image region, an image viewing direction, patient preparation, patient positioning, necessity of support devices for the patient.

For example, a visible patient property such as size and weight may be used for adapting the examination parameters, for example setting parameters for an X-ray tube such as dose pre-settings, and/or tube voltage. The image region may be individualized depending on the size of the patient, for example smaller regions for smaller patients, and larger regions for larger patients. If a disease such as scoliosis may be derived from the image data as a visible patient property, the patient positioning may be adapted. Further, if it is determined that the patient uses support devices and may not be able to stand, the patient preparation and patient position may be adapted accordingly for the examination.

According to a second aspect of the present invention a system for optimizing an examination is provided. The system comprises a user interface for displaying instructions to the patient for simulating the examination, an optical sensor configured to receive image data from the patient comprising information about at least one portion of the patient. Further the system comprises a processor configured to analyze the image data of the patient when the patient has been instructed by the instructions, determine visible patient properties based on the analyzed image data, wherein the processor is in communication with an external examination unit and the processor is configured to provide the visible patient properties to the external examination unit.

In particular, the processor may be configured to provide the visible patient properties via communication channel to the external examination unit for adapting the workflow of the planned examination. The system may be configured to carry out the method according to the exemplary embodiments herein. In particular, the processor of the system may be configured to perform the method steps as described in the various embodiments.

The present invention provides a system for optimizing an examination, wherein the system may comprise one or more processing devices, such as the processor, to carry out and/or control any of the steps of the method according to the present disclosure, particularly of the method claims.

According to an exemplary embodiment of the invention, the system may further comprise a handheld device comprising the user interface, the optical sensor and the processor, wherein the handheld device is configured to perform the method according to any of the exemplary embodiments via a software app. In particular, the handheld device may be a mobile communication unit, such as a mobile phone or a tablet. Therefore, the system allows to instruct and hence prepare the patient outside of the examination unit. For instance, the system allows to instruct the patient at home.

According to an exemplary embodiment, the visible patient properties may comprise a 3D model of at least one anatomy part of the patient and/or a mobility protocol of the patient. The visible patient properties may be properties relevant for the planned examination and which may differ from patient to patient, such that an individual adaptation may be necessary in the workflow of the examination.

The system may optionally comprise data connections to the external examination unit, data connections to imaging units, for example an X-ray system and its X-ray source, such that a data exchange between these elements may be possible. The workflow of the examination unit may be adapted based on these data exchanges.

According to an exemplary embodiment, the optical sensor may be at least one of a 2D camera, or a 3D camera, in particular a mobile phone camera attached to the user interface. This allows to use the inbuild hardware of the system for providing the elements for carrying out the method as described with the exemplary embodiments.

According to a third aspect of the present invention, a computer program product comprising instructions, when the program is executed by a computer, cause the computer to carry out the method as described herein with exemplary embodiments. In detail, the program product may cause the computer to carry out the method for optimizing an examination according to the first aspect of the present invention as described with any one of the embodiments herein.

The computer program product may be part of a computer program, but it can also be an entire program by itself. For example, the computer program product may be used to update an already existing computer program to get to the present invention.

The program may be stored on a computer readable medium. The computer readable medium may be seen as a storage medium, such as for example, a USB stick, a CD, a DVD, a data storage device, a hard disk, or any other medium on which a program element as described above can be stored.

For example, the method and/or the computer program product may be implemented as part of the operating system of the device. In that case, the vendor of the device provides the above functions to optimize an examination to all customers. Alternatively, the vendor may provide a switch in the system settings of the device where the user may have the option to switch off or customize the function. For instance, the method and/or the computer program product may be implemented as an App, for instance as part of the MR System in a hospital or as part of a portable X-ray system, and/or as part of a mobile phone for home use.

According to a further aspect of the invention, a computer-readable medium comprising instructions which, when executed by a computer, cause the computer to carry out the method of any one of the embodiments as described herein. In detail, the computer-readable medium may cause the computer to carry out the method for optimizing an examination according to the first aspect of the present invention as described with any one of the embodiments herein.

It has to be noted that embodiments of the invention have been described with reference to different subject matters. In particular, some embodiments have been described with reference to device/system type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the device type claims and features of the method type claims is considered as to be disclosed with this application.

It should be noted that the term "comprising" does not exclude other elements or steps and "a" or "an" does not exclude a plurality. Also, elements described in association with different embodiments may be combined. It should also be noted that reference signs in the claims should not be construed as limiting the scope of the claims.

### BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.
Fig. 1 illustrates schematically a system according to an exemplary embodiment of the invention.
Fig. 2 illustrates schematically the system according to an exemplary embodiment of the invention and external unit in communication with the system.
Fig. 3 illustrates schematically a flow diagram of the method according to an exemplary embodiment of the invention.

### DETAILED DESCRIPTION OF EMBODIMENTS

The illustrations in the drawings are schematic. It is noted that in different figures similar or identical elements are provided with the same reference signs.

Fig. 1 illustrates schematically a system 100 according to an exemplary embodiment of the invention. The system 100 comprises a user interface 104 for displaying instructions 109 to the patient 102 for simulating the examination, an optical sensor 105 configured to receive image data from the patient 102 comprising information about at least one portion 103 of the patient 102. Further, the system 100 comprises a processor configured to analyze the image data of the patient 102 when the patient has been instructed by the instructions 109, determine visible patient properties based on the analyzed image data, wherein the processor is in communication with an external examination unit and the processor is configured to provide the visible patient properties to the external examination unit. As can be seen in Fig. 1 the system 100 is illustrated as a mobile phone 101, which uses its optical sensor 105, the camera 105, for receiving image data of the patient 102. The patient 102 is illustrated schematically. The field of view 106 of the optical sensor 105 is illustrated, which field of view 106 captures in Fig. 1 the whole patient 102. It may also be possible that only one body part 103 is analyzed. The patient 102 may be observed via the optical sensor 105 and may prior or simultaneously be instructed via the user interface 104. For instructing the patient 102 an avatar 108 of the patient 102, or an image of the patient 102 is displayed. The instructions 109 are displayed as arrows indicating the direction for a desired position. Other images or indicators may be used for illustrating/displaying the instructions 109 via the display of the user interface 104. In Fig. 1 the arrows indicating the instructions 109 show that the ankle joints should be positioned more outwards. From the captured image data, a patient model 107 is derived, the patient model is illustrated in Fig. 1 as lines indicating the body parts such as legs and arms and as dots indicating the joints 111 between the different body parts. Further, audio features 110 are illustrated on the user interface 104, such that the patient 102 may also be instructed with audio content.

Fig. 2 illustrates schematically the system 100 according to an exemplary embodiment of the invention and external units in communication with the system 100. In particular, it is shown that the system 100 may be in external communication with the external examination unit 223 and that it may also be in communication with a server 226. The system 100 comprises the user interface 104, the optical sensor 105, at least one processor 221 and software application 222. In particular, the system is configured to run/perform the software application 222 which may be configured to perform the method as described with the exemplary embodiments herein. The system 100 is able to communicate with the external examination unit 223 and the server 226. The server may provide at least data from further software applications 227 a to c, from which the system 100 may receive further patient properties. On the other hand, it may be possible that the further software applications 227 a to c are implemented in the system 100 itself. The examination unit 223 comprises at least a processing unit for receiving the data from the system 100 and for analyzing the received data such that a workflow of the examination unit 224 can be adapted. Further, the examination unit 223 comprises an imaging unit 225, for example an X-ray imaging unit. The examination unit 223 may comprise further elements, which need to be adapted for the workflow, wherein these elements are not illustrated in Fig. 2.

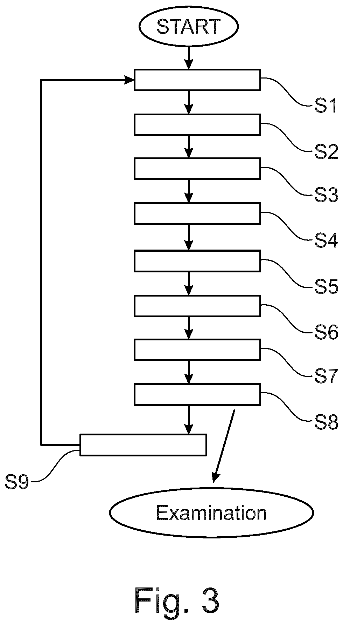

Fig. 3 illustrates schematically a flow diagram of the method according to an exemplary embodiment of the invention. The method may comprise the steps S1 to S9, wherein the method may not be limited to those steps, or further intermediate steps may be applicable. In Fig. 3 the start of the method is illustrated by step S1 displaying instructions to the patient 102. The following step S2 comprises instructing the patient via the user interface 104. The next step S3 comprises capturing images from the patient via the optical sensor 105, wherein this step may be performed simultaneously with the displaying of the instructions in step S1. In step S4 the image data is received by the processor, wherein the received image data comprises information about at least one portion of the patient. In step S5 the image data from the patient are analyzed by a processor, when the patient has been instructed. Afterwards, in step S6 the visible patient properties are determined based on the analyzed image data. Further, in step S7 the visible patient properties are provided to an external examination unit 223. In step S8 the workflow of the examination is adapted based on the determined visible patient properties. Moreover, in Fig. 3 it is illustrated that in step S9 further information may be received, such as further information about the workflow, or a feedback form the examination unit, which may require a repeated instruction of the patient. Step S9 may be optional but if further information may be received the method may be repeated and may start with step 1 again. If no further information is received and the workflow can be adapted successfully and sufficiently, the examination of the patient may be sufficiently prepared and may be carried out.

### LIST OF REFERENCE SIGNS:

- 100: system
- 101: mobile phone
- 102: patient
- 103: body part
- 104: user interface
- 105: optical sensor
- 106: field of view
- 107: patient model
- 108: patient avatar
- 109: instruction
- 110: audio
- 111: model j oint of patient
- 221: processor
- 222: software application
- 223: examination unit
- 224: processing unit
- 225: imaging unit
- 226: server
- 227: software application
- S1 to S9: method steps

## Claims

1. A computer-implemented method for optimizing an examination, wherein the method comprises the steps of
instructing the patient by displaying instructions via a user interface for simulating the examination;
receiving image data comprising information about at least one portion of the patient via an optical sensor;
analyzing, by a processor, the image data from the patient when the patient has been instructed;
determining visible patient properties based on the analyzed image data;
providing the visible patient properties to an external examination unit;
adapting a workflow of the examination based on the determined visible patient properties.

2. The method according to claim 1, further comprising the steps of:
applying a 3D anatomical model to the image data;
wherein the step of determining patient properties further comprises:
adapting the 3D anatomical model to the patient using the determined visible patient properties for generating a patient specific 3D anatomical model wherein the patient specific 3D anatomical model is provided to the external examination unit.

3. The method according to claim 1 or 2,
wherein the step of determining patient properties further comprises generating a mobility protocol of the patient based on the analyzed image data and/or the visible patient properties,
wherein the mobility protocol comprises information about an overall mobility of the patient derived from the image data and/or the visible patient properties received during performing the instructions by the patient.

4. The method according to any one of the preceding claims,
wherein the step of determining patient properties further comprises determining at least one of a size of the patient, a weight of the patient, a disease of the patient, or a physical malfunction of the patient from the image data.

5. The method according to any one of the preceding claims, wherein the method further comprises the steps of:
providing feedback to the patient via the user interface, whether the instructions have been performed correctly,
displaying the feedback via the user interface.

6. The method according to any one of the preceding claims, further comprising the step of
receiving an input from the patient about non-visible patient properties,
wherein the non-visible patient properties are at least one of an age of the patient, a patient language, date of the examination, time of the examination, or a disease of the patient.

7. The method according to any one of the preceding claims,
wherein the processor is in communication with at least one software app, wherein the method comprises the further step of receiving visible and/or non-visible patient properties from the software app.

8. The method according to any one of the preceding claims, further comprising the step of
receiving information from the external examination unit for further instructions to be displayed to the patient via the user interface, after the workflow of the examination has been adapted based on the previously determined visible patient properties,
displaying the further instructions to the patient,
receiving further image data comprising information about the at least one portion of the patient via the optical sensor;
repeat analyzing the image data from the patient when displaying the instructions;
determining further visible patient properties based on the analyzed image data;
providing the further visible patient properties to the external examination unit;
repeat adapting of the workflow of the examination based on the determined further visible patient properties.

9. The method according to any one of the preceding claims,
wherein the instruction comprises:
displaying single images for positioning the at least one portion of the patient in a position for the examination, and/or
displaying image sequences for performing a movement of the at least one portion of the patient for the examination.

10. The method according to any of the preceding claims,
wherein the step of adapting a workflow of the examination further comprises adapting examination parameters,
wherein the examination parameters are at least one of an imaging parameter, an image region, an image viewing direction, patient preparation, patient positioning, necessity of support devices for the patient.

11. A system for optimizing an examination, wherein the system comprises:
a user interface for displaying instructions to the patient for simulating the examination;
an optical sensor configured to receive image data from the patient comprising information about at least one portion of the patient;
a processor configured to:
analyze the image data of the patient when the patient has been instructed by the instructions,
determine visible patient properties based on the analyzed image data,
wherein the processor is in communication with an external examination unit and the processor is configured to provide the visible patient properties to the external examination unit.

12. The system according to the preceding claim 11, wherein the system further comprises:
a handheld device comprising the user interface, the optical sensor and the processor, wherein the handheld device is configured to perform the method of the claims 1 to 10 via a software app.

13. The system according to the preceding claims 11 or 12,
wherein the visible patient properties comprise a 3D model of at least one anatomy part of the patient and/or a mobility protocol of the patient.

14. A computer program product comprising instructions which, when the program is executed by a computing system, cause the computing system to carry out the method of any of claims 1 to 10.

15. A computer readable medium having stored thereon instructions which, when the program is executed by a computing system, cause the computing system to carry out the method of any of claims 1 to 10.
